# EUROPEAN PATENT SPECIFICATION

(11) **EP 2 922 558 B1**
(45) Date of publication and mention of the grant of the patent: **24.08.2016**
(21) Application number: 13783916.3
(22) Date of filing: 29.10.2013
(51) Int. Cl.: A61K 36/28, A61K 36/82, A61P 3/10, A61K 31/198, A61K 31/353

(54) **COMPOSITION COMPRISING THEANINE AND AN EXTRACT OF INULA RACEMOSA**
ZUSAMMENSETZUNG MIT THEANIN UND EINEM EXTRAKT AUS INULA RACEMOSA
COMPOSITION COMPRENANT DE LA THÉANINE ET UN EXTRAIT D'INULA RACEMOSA

(30) Priority: 21.11.2012 EP 12193516
(43) Date of publication of application: 30.09.2015
(73) Proprietor: Unilever N.V., 3013 AL Rotterdam (NL); Unilever PLC, London, Greater London EC4Y 0DY (GB)
(72) Inventor: BANERJEE, Gautam, Bangalore 560 066 (IN); KADAMKODE, Vinitha, Bangalore 560 066 (IN); KALATHIL, Ramitha, Bangalore 560 066 (IN); MAJUMDER, Suman, West Bengal 700 019 (IN)
(74) Representative: Warner, Guy Jonathan
(86) International application number: PCT/EP2013/072620
(87) International publication number: WO 2014/079665

(56) References cited:
- MUKHERJEE P K ET AL: "Leads from Indian medicinal plants with hypoglycemic potentials", JOURNAL OF ETHNOPHARMACOLOGY, ELSEVIER SCIENTIFIC PUBLISHERS LTD, IE, vol. 106, no. 1, 15 June 2006 (2006-06-15) , pages 1-28, XP025085618, ISSN: 0378-8741, DOI: 10.1016/J.JEP.2006.03.021 [retrieved on 2006-06-15]
- ABEYWICKRAMA K R W ET AL: "Oral hypoglycaemic, antihyperglycaemic and antidiabetic activities of Sri Lankan Broken Orange Pekoe Fannings (BOPF) grade black tea (L.) in rats", JOURNAL OF ETHNOPHARMACOLOGY, ELSEVIER SCIENTIFIC PUBLISHERS LTD, IE, vol. 135, no. 2, 8 February 2011 (2011-02-08), pages 278-286, XP028203413, ISSN: 0378-8741, DOI: 10.1016/J.JEP.2011.02.035 [retrieved on 2011-03-09]
- ZEGGWAGH N A ET AL: "Study of hypoglycaemic and hypolipidemic effects of Inula viscosa L. aqueous extract in normal and diabetic rats", JOURNAL OF ETHNOPHARMACOLOGY, ELSEVIER SCIENTIFIC PUBLISHERS LTD, IE, vol. 108, no. 2, 24 November 2006 (2006-11-24), pages 223-227, XP027939761, ISSN: 0378-8741 [retrieved on 2006-11-24]
- CHATURVEDI P ET AL: "STIMULATION OF INSULIN SENSITIVITY BY INULA-RACEMOSA ROOT EXTRACT", THE FASEB JOURNAL, FEDERATION OF AMERICAN SOCIETIES FOR EXPERIMENTAL BIOLOGY, BETHESDA; US, vol. 5, no. 4, 1 November 1991 (1991-11-01), page A825, XP009186999, ISSN: 0892-6638
- T N SINGH ET AL: 'Management of diabetes mellitus (prameha) with inula racemosa and cinnamomum tamala' ANCIENT SCIENCE OF LIFE vol. 5, no. 1, 01 July 1985, IN, pages 9 - 16, XP055225202 ISSN: 0257-7941

## Description

### Field of the Invention

The present invention relates to a composition comprising theanine and an extract of *Inula racemosa.* The invention further relates to a process for obtaining such.

### Background of the invention

Adiponectin is a protein circulating in the human body (in blood). It is secreted from adipose tissue. The levels of adiponectin circulating in the blood decrease in individuals with pre-diabetes and type 2 diabetes. In people suffering from pre-diabetes and type 2 diabetes the insulin responsive tissues in the body like skeletal muscle, adipose tissue, and liver develop insulin resistance (i.e. a decreased response towards insulin), and such individuals may eventually develop clinical hyperglycemia.

Adiponectin is a positive regulator of insulin sensitivity: it improves the response of tissues towards insulin, and thus helps to maintain blood glucose levels in the body. Hence any natural ingredient which induces adiponectin secretion helps in increasing the insulin sensitivity, and this in turn is good for blood glucose control.

*Inula racemosa* is a herb known in India for its use in treatment of diabetics, and is also known under the Ayurvedic name Pushkarmool. Pushkarmool then usually refers to an extract of the roots of the herb *inula racemosa.* Neither the exact composition of such extract has been clarified so far, nor its mode of action in the prevention or treatment of diabetes 2 or pre-diabetes.

The natural ingredient theanine (also known as gamma-glutamylethylamide or 5-N-ethyl-glutamine) is reported for use in treating people with diabetes or at risk thereof. Theanine is present in green tea and some other vegetable extracts. The precise way in which theanine would support treatment or prevention of diabetes 2 or pre-diabetes is unknown.

XP009186999 (Chaturvedi et.al., 1991) discloses that *Inula racemosa* is a plant belonging to the Compositae family and the root of this plant is used as a medicine for diabetes in accordance with Ayurveda. Inula has strong hypoglycaemic response and it is said to be due to regulation of insulin receptor sensitivity.

In a journal article published in Ancient Science of Life, Vol no. V No.1 July 1985 (Singh et. al,) discloses that *Inula racemosa* provides good diabetes control and that the response of *Inula racemosa* is better than *C.tamala*.

Multiple drugs have been developed so far and remain to be developed for use in the treatment of diabetes type 2 or the progression of pre-diabetes to diabetes type 2. Despite some success, there is also the desire for compositions that can be used in foods by consumers to reduce the risk of developing diabetes type 2 and/or pre-diabetes.

### Summary of the Invention

It is an object of the present invention to provide an edible composition that can be used for stimulating adiponectin release by the human body and/or that can increase the level of adiponectin circulating in the bloodstream of humans.

It has now been found that such can be achieved by an edible composition comprising theanine and an extract of *inula racemosa,* which does not contain green tea or extracts of green tea.

The invention further relates to an edible composition comprising theanine and an extract of *inula racemosa*, which optionally comprises EGCG, wherein, if the mixture comprises EGCG, the weight ratio theanine / EGCG is less than 5, preferably less than 3, more preferably less than 2.

The invention also relates to a process for the preparation of a composition comprising an extract of *inula racemosa* and theanine, which process comprises obtaining dry ground root of Pushkarmool, extracting such with a liquid composition comprising a lower alcohol in an amount of 50-99 wt% in water, drying the obtained extract, blending the so-obtained dry extract with theanine such that theanine and the extract of *inula racemosa* are present in a weight ratio of from 1 : 0.1 to 1 : 50.

The invention further relates to the use of a composition comprising theanine and an extract of *inula racemosa* wherein theanine and the extract of *inula racemosa* are present in a weight ratio of theanine : extract of *inula racemosa* from 1 : 0.1 to 1 : 50, in the manufacture of a food or medicament to stimulate insulin sensitivity and/or for treatment of pre-diabetes and/or for treatment of diabetes type 2.

The invention further relates to a composition comprising theanine and an extract of *inula racemosa* wherein theanine and the extract of *inula racemosa* are present in a weight ratio of theanine : extract of *inula racemosa* from 1 : 0.1 to 1 : 50, for use in the stimulation of insulin sensitivity and/or for treatment of pre-diabetes and/or for treatment of diabetes type 2

### Detailed description of the invention

"Theanine"(also known under the names of 2-Amino-4-(ethylcarbamoyl)butyric acid; or *N*-ethyl-L-glutamine; or gamma-glutamylethylamide or 5-N-ethyl-glutamine herein is to be understood the L-amino acid form, known as L-theanine, and herein encompasses the edible salts thereof.

*Inula racemosa* (also spelled as *innula racemosa*) is a species of an ornamental plant of the Asteraceae family. Inula racemosa grows in the temperate and alpine western Himalayas, and it is common in Kashmir, and known under the Ayurvedic name "Pushkarmool". "Extract of *inula racemosa*" herein is to be understood as a composition obtainable by extracting roots of such plants or parts of such roots with a liquid comprising water and a lower alcohol (C1-C6). Herein, "extract of *inula racemosa*" is the same as "*inula racemosa* extract".

"Tea" for the purposes of the present invention means material from *Camellia sinensis* var. *sinensis* and/or *Camellia sinensis* var. *assamica*. "Green tea" refers to substantially unfermented tea. "Black tea" refers to substantially fermented tea. "Oolong tea" refers to partially fermented tea. Fermentation refers to the oxidative and hydrolytic process that tea undergoes when certain endogenous enzymes and substrates are brought together, e.g., by mechanical disruption of the cell by maceration of the leaves. During this process colourless catechins in the leaves are converted to a complex mixture of yellow and orange to dark-brown polyphenolic substances. Fresh tea leaves are subjected to an enzyme deactivation step to prevent fermentation if they are to be processed to yield a green tea product.

"EGCG" herein is to be understood as epigallocatechin gallate, also known as epigallocatechin-3-gallate. EGCG is present both in green tea as well as in black tea, but it is far more abundant in green tea (usually 5-10 times more).

Extracts of Pushkarmool or *inula racemosa* are known in India for its use in treatment of diabetics. Such extract then usually refers to an extract of the roots of the herb *inula racemosa*. Neither the exact composition of such extract has been clarified so far, nor its mode of action in the prevention or treatment of diabetes 2 or pre-diabetes.

Theanine is a naturally occurring compound (also available from chemical origin) and such is reported for use in treating people with diabetes or at risk thereof. Its mechanism is yet unknown. Theanine occurs in (extracts of) tea, both in green tea as well as black tea, in fairly similar amounts.

It was found by the present inventors that the combination of theanine and an extract of *inula racemosa* shows beneficial effect on adiponectin secretion (as determined by an assay using differentiated 3T3L1 mouse adipocytes, which is believed to be predictive of adiponectin secretion or stimulation in humans), and such being more so than the effects of theanine and the extract of *inula racemosa* on its own, and even more so than the additive effect on adiponectin secretion by these individual compounds. In other words: it was found that theanine and an extract of *inula racemosa* seem to have synergistic action on stimulation of adiponectin secretion in humans. Hence, the present invention relates to combinations of theanine and an extract of *inula racemosa*, as an active cocktail, and its use in combination in foods like e.g. black tea and starch- or sugar-rich foodstuffs or for addition to such, e.g. as a seasoning.

In the compositions, uses, processes and methods according to the present invention it is preferred, especially for good synergistic action, that theanine and the extract of *inula racemosa* are present in a weight ratio of theanine : extract of *inula racemosa* from 1 : 0.1 to 1 : 50, and more preferably in a weight ratio of from 1 : 0.5 to 1 : 20.

Theanine and the extract of *inula racemosa* according to the present invention may be used as such for addition to foods, or be part of a seasoning (e.g. a table top seasoning further containing salt and/or herbs or spices), or in a composition further comprising black tea and/or black tea extracts, or in a composition further comprising digestible carbohydrates in an amount of from 40 to 99% by weight based on the total composition. The latter may be e.g. flour such as atta flour for making chapatti, or other staple food rich in digestible carbohydrates, as (regular) consumers of such food may benefit most from the effect on adiponectin secretion of the compositions according to the present invention.

A composition containing both the extract of *inula racemosa* and theanine according to the present invention may suitably be formulated as a seasoning, e.g. a table top seasoning. Such seasoning can e.g. be added to digestible starch-rich foods such as rice or dishes containing rice, to manage (at least in part) the effects caused by ingestion of large amounts of quickly digestible starch. Hence, the invention further relates to the compositions according to the invention (i.e. containing the extract of inula racemosa and theanine) further comprising 30-95% of salt, by weight based on the total composition. Next to salt, such seasoning may further comprise e.g. 10-50% by weight herbs and/or spices.

In the compositions, uses, methods and processes of the present invention it is preferred that the *inula racemosa* extract is an extract of *inula racemosa* root or parts thereof (as the roots are believed to contain (most) of the actives). Herein, it is preferred that the extracts, from roots or other parts of the *inula racemosa,* are obtainable by extracting said vegetable matter with a liquid composition comprising a lower alcohol in an amount of 50-99 wt% (preferably such lower alcohol in an amount of 65-90 wt%) in water. In the extraction, the lower alcohol is preferably an alcohol having from 1 to 6 carbon atoms (i.e. a C1-C6 alcohol, preferably an alkanol), most preferably being ethanol.

The invention further relates to a process for the preparation of a composition comprising an extract of *inula racemosa* and theanine, which process comprises obtaining dry ground root of Pushkarmool, extracting such with a liquid composition comprising a lower alcohol (preferably such lower alcohol is an alkanol having 1-6 carbon atoms, with ethanol being preferred) in an amount of 50-99 wt% in water, drying the obtained extract, blending the so-obtained dry extract with theanine such that theanine and the extract of *inula racemosa* are present in a weight ratio of from 1 : 0.1 to 1 : 50 (and more preferably in a weight ratio of from 1 : 0.5 to 1 : 20). In such process, it is preferred, for a good extraction, that the extraction of the dry ground root of Pushkarmool comprises soaking such dry ground root in said liquid composition comprising the lower alcohol for a period of 4 to 48 hours at a temperature of 5 to 40°C, followed by heating to a temperature above 40°C for 1 to 12 hours, followed by removing insoluble matter. It is the liquid obtained after removal of the insoluble matter that contains the actives. Such liquid can be used as such, but is usually concentrated. Such concentration can be effected by any suitable means. For obtaining a dry ingredient, the concentration involves drying (i.e. removal of water, alcohol and optional other solvents.

### Example

The potential stimulating effect of theanine and an extract of *inula racemosa* (jointly and apart from eachother) on adiponectin secretion in humans was assessed by an assay.

### Materials

### Theanine

The theanine used was purchased from Taiyo green tea products (Japan) with trade name Suntheanine 100% pure L-isomer Theanine.

### Extract of inula racemosa

The extract of *inula racemosa* that was used was obtained by subjecting a commercial sample of dry inula powder. This sample was purchased from a local trader from Bangalore (Channabasappa) with trade name Pushkarmool. to the following process:
- Soaking (overnight, approx. 16-18 hours) 100 g of the dry commercial *inula racemosa* powder in 800 ml of a mixture comprising 75% ethanol and 25% water (wt %);
- Heating such soaked powder to 55-60°C for 2 hours;
- Cooling down to 35-40°C, and filtering off the residue to obtain a clear filtrate;
- Concentrating the filtrate using a rotary evaporator to a dry powder, which was stored at 4°C until usage.

### Adiponectin assay protocol

- 3T3L1 (mouse preadipocytes) were plated on a 96 well plate at a density of 3*10³ cells per well;
- After 5 days of post confluency (a condition where the cells form a uniform layer) the cells were differentiated (a process where premature adipocytes are transformed to mature adipocytes) in differentiation medium for 10 days;
- On the 11^{th} day the matured adipocytes were treated with non cytotoxic concentrations (concentrations stated below) of the *inula racemosa* extract and theanine;
- The cells were incubated for 48 hrs and supernatants were collected and stored. They were analysed to detect level of Adiponectin by ELISA;
- The efficacy of the test ingredient was evaluated with respect to the untreated control (media control);
- If any of the tested combinations of *inula racemosa* extract and theanine on increased adiponectin level was larger in a statistically significant way than the additive effect or individual components, then the particular combination is considered to be synergistic.

### Quantification of Total Polyphenol in 75% aqueous ethanolic extract of inula racemosa

Total Polyphenol content (TPP) in samples of *inula racemosa* was quantified using Folin-Ciocalteu reagent (ISO 14502-1:2005(E). TPP was expressed as equivalent of Gallic acid (result: 1 %).This indicates that the polyphenol content of the 75% aqueous ethanolic extract is significantly low.

Quantification of the ethanolic extract shows that the allantolactones present in 75% aqueous ethanolic extract by IR (Infrared Spectroscopy) is 2.5 - 3 %. (Ref. published by Unilever R & D, BL Phytochem. Anal. 2012, 23, 171-176).

Based on this, it is believed that the extract of *inula racemosa* is rich in other neutral compounds like triterpene glycosides which may be responsible for the bio-activity.

### In-vitro effects of inula racemosa extract and theanine, separately

To investigate the efficacy of inula racemosa and theanine in increasing adiponectin secretion from 3T3L1, the cells were treated with varying concentrations of *inula racemosa* (as obtained set out herein in the Materials section) and theanine in cell culture medium. The % of adiponectin from control cells were taken as hundred, upon which the treatment values were calculated. From the results in Table 1 it is evident that (an alcoholic/aqueous extract of) inula racemosa and theanine increased adiponectin levels in an in-vitro study in a dose response way.

**Table 1**

| Wt% of inular. extract or theanine | Control | 0.00001 % | 0.00002 % | 0.00005 % | 0.0001% | 0.0005% | 0.001% |
|---|---|---|---|---|---|---|---|
| Inula | 100.00 | 103.6+/- 4.1 | 110.9+/- 7.6 | 117.0+/- 4.0 | 130.7+/- 4.3 | 137.1+/- 7.6 | 156.9+/ -7.9 |
| p value | | p <0.3 | p <0.05 | p <0.05 | p <0.05 | p <0.05 | p <0.05 |
| Theanine | 100.00 | 106.7+/- 7.4 | 107.0+/- 7.0 | 136.7+/- 7.3 | 141.7+/- 1.6 | 145.8+/- 8.2 | 156.0+/ -8.5 |
| p value | | p <0.05 | p < 0.08 | p <0.05 | p <0.05 | p <0.05 | p <0.05 |

### In-vitro effects of inula racemosa extract and theanine jointly.

A range of combination varying amounts of *inula racemosa* extract and theanine were investigated on its joint ability for adiponectin secretion. The combinations used were (weight ratio's theanine : *inula racemosa* extract): 100:1 (A), 100:2 (B), 100:10 (C), 100:50 (D), 100:500(E), 100:1000 (F), 100:5000 (G), 100:10,000 (H). This was achieved via dissolution of the components in aqueous stock solutions. The main stock of theanine (10%) was made in cell culture medium, and the main stock of Inula ethanolic extract was made in 75% ethanol, and both theanine and Inula extract were serially diluted in cell culture medium to obtain the desired concentration.

From Table 2 it is clear that the *inula racemosa* extract and theanine in the ratios under C to G in table 2 (100:10 to 100:5000) of theanine : *inula racemosa* extract exhibit a significant synergistic effect on adiponectin secretion in-vitro.

**Table 2**

| | Theanine : inula racemosa extract | Combination value ( % Adiponectin) | Additive Value (% Adiponectin) | • Value* |
|---|---|---|---|---|
| Control | 0 | 100 | 100 | - |
| A | 100:1 (0.001 %+0.00001%) | 122+/- 4.9 | 111 | • <0.1 |
| B | 100:2 (0.0005%+0.00001%) | 154+/- 9.8 | 146 | • <0.1 |
| C | 100:10 (0.0001%+0.00001%) | 179+/- 5.8 | 150 | • <0.05 |
| D | 100:50 (0.00002%+0.00001%) | 219+/- 2.2 | 160 | • <0.05 |
| E | 100:500 (0.00001%+0.00005%) | 158+/- 1.5 | 141 | • <0.05 |
| F | 100:1000 (0.00001%+0.0001%) | 155+/- 4.0 | 146 | • <0.05 |
| G | 100:5000 (0.00001%+0.0005%) | 160+/- 4.2 | 150 | • <0.05 |
| H | 100:10,000 (0.00001%+0.001%) | 176+/- 3.0 | 160 | • <0.08 |

| | | | | |
|---|---|---|---|---|
| *Significance calculated with respect to the additive values | | | | |

### Conclusion

Theanine and an extract of inula racemosa in ratios of theanine : *inula racemosa* extract from 100:10 to 100:5000 (equals 1 : 0.1 to 1 : 50) of may be incorporated beneficially in various foods and beverages format as necessary for prevention against diabetes, as this combination in such ratio's is a synergistic combination as evidenced by the in-vitro assay used.

## Claims

1. Edible composition comprising theanine and an extract of *inula racemosa*, which does not contain green tea or extracts of green tea.

2. Edible composition comprising theanine and an extract of *inula racemosa*, which optionally comprises EGCG, wherein, if the mixture comprises EGCG, the weight ratio theanine / EGCG is less than 5, preferably less than 3, more preferably less than 2.

3. Composition according to claim 1 or 2, wherein the *inula racemosa* extract is an extract of *inula racemosa* root or parts thereof.

4. Composition according to any of the preceding claims, wherein the extract of *inula racemosa* is obtainable by extracting said vegetable matter with a liquid composition comprising a lower alcohol in an amount of 50-99 wt% in water.

5. Composition according to any of the preceding claims, wherein theanine and the extract of *inula racemosa* are present in a weight ratio of theanine : extract of *inula racemosa* from 1 : 0.1 to 1 : 50.

6. Composition according to any of the preceding claims, further comprising black tea and/or black tea extracts.

7. Composition according to any of claims 1-5, further comprising digestible carbohydrates in an amount of from 40 to 99% by weight based on the total composition.

8. Composition according to any of claims 1-5, further comprising 30-95% of salt, by weight based on the total composition.

9. Process for the preparation of a composition comprising an extract of *inula racemosa* and theanine, which process comprises obtaining dry ground root of Pushkarmool (*Inula racemosa*), extracting such with a liquid composition comprising a lower alcohol in an amount of 50-99 wt% in water, drying the obtained extract, blending the so-obtained dry extract with theanine such that theanine and the extract of *inula racemosa* are present in a weight ratio of from 1 : 0.1 to 1 : 50.

10. Process according to claim 9, wherein the lower alcohol is ethanol.

11. Process according to claim 9 or 10, wherein the concentration of the lower alcohol is 65-90%.

12. Process according to any of claims 9 to 11, wherein the extraction the dry ground root of Pushkarmool comprises soaking such dry ground root in said liquid composition comprising the lower alcohol for a period of 4 to 48 hours at a temperature of 5 to 40°C, followed by heating to a temperature above 40°C for 1 to 12 hours, followed by removing insoluble matter.

13. Process according to claim 10, wherein the liquid obtained after removal of the insoluble is concentrated.

14. Use of a composition comprising theanine and an extract of *inula racemosa* wherein theanine and the extract of *inula racemosa* are present in a weight ratio of theanine : extract of *inula racemosa* from 1 : 0.1 to 1 : 50, in the manufacture of a food or medicament to stimulate insulin sensitivity and/or for treatment of pre-diabetes and/or for treatment of diabetes type 2.

15. A composition comprising theanine and an extract of *inula racemosa* wherein theanine and the extract of *inula racemosa* are present in a weight ratio of theanine : extract of *inula racemosa* from 1 : 0.1 to 1 : 50, for use in the stimulation of insulin sensitivity and/or for treatment of pre-diabetes and/or for treatment of diabetes type 2.

## Patentansprüche

1. Essbare Zusammensetzung, umfassend Theanin und einen Extrakt von *inula racemosa*, der keinen grünen Tee oder Extrakte von grünem Tee enthält.

2. Essbare Zusammensetzung, umfassend Theanin und einen Extrakt von *inula racemosa*, der optional EGCG umfasst, worin, wenn die Mischung EGCG umfasst, das Gewichtsverhältnis Theanin/EGCG weniger als 5, vorzugsweise weniger als 3, bevorzugter weniger als 2 ist.

3. Zusammensetzung nach Anspruch 1 oder 2, wobei der *inula racemosa*-Extrakt ein Extrakt von *inula racemosa*-Wurzel oder Teilen davon ist.

4. Zusammensetzung nach irgendeinem der vorhergehenden Ansprüche, wobei der Extrakt von *inula racemosa* durch Extrahieren des pflanzlichen Materials mit einer flüssigen Zusammensetzung erhältlich ist, die einen niederen Alkohol in einer Menge von 50-99 Gew.-% in Wasser umfasst.

5. Zusammensetzung nach irgendeinem der vorhergehenden Ansprüche, worin Theanin und der Extrakt von *inula racemosa* in einem Gewichtsverhältnis von Theanin : Extrakt von *inula racemosa* von 1:0,1 bis 1:50 vorliegen.

6. Zusammensetzung nach irgendeinem der vorhergehenden Ansprüche, die des Weiteren schwarzen Tee und/oder Extrakte von schwarzem Tee umfasst.

7. Zusammensetzung nach irgendeinem der Ansprüche 1-5, die des Weiteren verdauliche Kohlenhydrate in einer Menge von 40 bis 99 Gew.-%, bezogen auf die gesamte Zusammensetzung, umfasst.

8. Zusammensetzung nach irgendeinem der Ansprüche 1-5, die des Weiteren 30-95 Gew.-% Salz, bezogen auf die gesamte Zusammensetzung, umfasst.

9. Verfahren zur Herstellung einer Zusammensetzung, umfassend einen Extrakt von *inula racemosa* und Theanin, wobei das Verfahren umfasst Erhalten von trockener gemahlener Wurzel von Pushkarmool (*inula racemosa*), Extrahieren solches mit einer flüssigen Zusammensetzung, die einen niederen Alkohol in einer Menge von 50-99 Gew.-% in Wasser enthält, Trocknen des erhaltenen Extrakts, Vermischen des so erhaltenen trockenen Extrakts mit Theanin derartig, dass Theanin und der Extrakt von *inula racemosa* in einem Gewichtsverhältnis von 1:0,1 bis 1:50 vorliegen.

10. Verfahren nach Anspruch 9, wobei der niedere Alkohol Ethanol ist.

11. Verfahren nach Anspruch 9 oder 10, wobei die Konzentration des niederen Alkohols 65-90% beträgt.

12. Verfahren nach irgendeinem der Ansprüche 9 bis 11, wobei die Extraktion der trockenen gemahlenen Wurzel von Pushkarmool umfasst das Einweichen derartiger trockener gemahlener Wurzel in der flüssigen Zusammensetzung, die den niederen Alkohol umfasst, während einer Zeitdauer von 4 bis 48 Stunden bei einer Temperatur von 5 bis 40°C, gefolgt von Erhitzen auf eine Temperatur oberhalb von 40°C während 1 bis 12 Stunden, gefolgt vom Entfernen unlöslichen Materials.

13. Verfahren nach Anspruch 10, wobei die Flüssigkeit, die nach dem Entfernen des unlöslichen Materials erhalten wurde, konzentriert wird.

14. Verwendung einer Zusammensetzung, umfassend Theanin und einen Extrakt von *inula racemosa*, wobei das Theanin und der Extrakt von *inula racemosa* in einem Gewichtsverhältnis von Theanin : Extrakt von *inula racemosa* 1:0,1 bis 1:50 vorliegen, zur Herstellung eines Lebensmittels oder eines Medikaments, um Insulinempfindlichkeit zu stimulieren und/oder zur Behandlung von Prädiabetes und/oder zur Behandlung von Typ 2-Diabetes.

15. Eine Zusammensetzung, umfassend Theanin und einen Extrakt von *inula racemosa*, wobei das Theanin und der Extrakt von *inula racemosa* in einem Gewichtsverhältnis von Theanin : Extrakt von *inula racemosa* 1:0,1 bis 1:50 vorliegen, zur Verwendung bei der Stimulation von Insulin-Empfindlichkeit und/oder zur Behandlung von Prädiabetes und/oder zur Behandlung von Typ 2-Diabetes.

## Revendications

1. Composition comestible comprenant de la théanine et un extrait d'*inula racemosa*, qui ne contient pas de thé vert ou d'extraits de thé vert.

2. Composition comestible comprenant de la théanine et un extrait d'*inula racemosa*, qui comprend facultativement de l'EGCG, dans laquelle, si le mélange comprend de l'EGCG, le rapport pondéral théanine/EGCG est inférieur à 5, de préférence inférieur à 3, plus préférablement inférieur à 2.

3. Composition selon la revendication 1 ou 2, dans laquelle l'extrait d'*inula racemosa* est un extrait de racine d'*inula racemosa* ou de parties de celle-ci.

4. Composition selon l'une quelconque des revendications précédentes, dans laquelle l'extrait d'*inula racemosa* peut être obtenu par extraction de ladite matière végétale avec une composition liquide comprenant un alcool inférieur en une quantité de 50 à 99 % en poids dans l'eau.

5. Composition selon l'une quelconque des revendications précédentes, dans laquelle la théanine et l'extrait d'*inula racemosa* sont présents en un rapport pondéral théanine/extrait d'*inula racemosa* de 1/0,1 à 1/50.

6. Composition selon l'une quelconque des revendications précédentes, comprenant en outre du thé noir et/ou des extraits de thé noir.

7. Composition selon l'une quelconque des revendications 1 à 5, comprenant en outre des glucides assimilables en une quantité de 40 à 99 % en poids sur la base de la composition totale.

8. Composition selon l'une quelconque des revendications 1 à 5, comprenant en outre de 30 à 95 % de sel, en poids sur la base de la composition totale.

9. Procédé de préparation d'une composition comprenant un extrait d'*inula racemosa* et de la théanine, lequel procédé comprend l'obtention de racine broyée sèche de Pushkarmool (*Inula racemosa*), l'extraction de celle-ci avec une composition liquide comprenant un alcool inférieur en une quantité de 50 à 99 % en poids dans l'eau, le séchage de l'extrait obtenu, le mélange de l'extrait sec ainsi obtenu avec de la théanine de sorte que la théanine et l'extrait d'*inula racemosa* soient présents en un rapport pondéral de 1/0,1 à 1/50.

10. Procédé selon la revendication 9, dans lequel l'alcool inférieur est l'éthanol.

11. Procédé selon la revendication 9 ou 10, dans lequel la concentration de l'alcool inférieur est de 65 à 90%.

12. Procédé selon l'une quelconque des revendications 9 à 11, dans lequel l'extraction de la racine broyée sèche de Pushkarmool comprend le trempage d'une telle racine broyée sèche dans ladite composition liquide comprenant l'alcool inférieur pendant une période de 4 à 48 heures à une température de 5 à 40 °C, suivi du chauffage à une température supérieure à 40 °C pendant 1 à 12 heures, suivi de l'élimination de la matière insoluble.

13. Procédé selon la revendication 10, dans lequel le liquide obtenu après élimination de la matière insoluble est concentré.

14. Utilisation d'une composition comprenant de la théanine et un extrait d'*inula racemosa* dans laquelle la théanine et l'extrait d'*inula racemosa* sont présents en un rapport pondéral théanine/extrait d'*inula racemosa* de 1/0,1 à 1/50, dans la fabrication d'un aliment ou d'un médicament permettant de stimuler la sensibilité à l'insuline et/ou destiné au traitement du pré-diabète et/ou destiné au traitement du diabète de type 2.

15. Composition comprenant de la théanine et un extrait d'*inula racemosa* dans laquelle la théanine et l'extrait d'*inula racemosa* sont présents en un rapport pondéral théanine/extrait d'*inula racemosa* de 1/0,1 à 1/50, pour utilisation dans la stimulation de la sensibilité à l'insuline et/ou destinée au traitement du pré-diabète et/ou destinée au traitement du diabète de type 2.
